# EUROPEAN PATENT APPLICATION

(11) **EP 2 926 894 A1**
(43) Date of publication of application: **07.10.2015**
(21) Application number: 14162698.6
(22) Date of filing: 31.03.2014
(51) Int. Cl.: B01J 13/10, B01J 13/20

(54) **Improvements in or relating to organic compounds**

(71) Applicant: Givaudan SA, 1214 Vernier (CH)
(72) Inventor: Bone, Stephane, 76750 Estouteville Ecalles (FR); Fadel, Addi, 75016 Paris (FR); Geffroy, Cedric, 86180 Buxerolles (FR)

(57) **Abstract**

A microcapsule composition consisting essentially of core material enclosed in a shell, wherein the shell comprises a complex coacervate formed from at least two oppositely charged colloids, one of which is a protein, and wherein the protein is cross linked to a hardening agent by amide groups.

## Description

The present invention is concerned with a microcapsule composition of the type consisting of a plurality of microcapsules, each comprising a core material encased or entrapped in a shell formed of a complex coacervate, a method of forming same, and the use of said microcapsule composition as delivery vehicle for active materials, in particular fragrance and flavour ingredients.

Microencapsulation is a process of encasing tiny droplets of a substance in protective coatings. This is done to assist in the storage, handling or controlling the release of the encapsulated substance. There are many techniques of forming microcapsules. One commonly employed technique is known as complex coacervation.

Coacervation is a process whereby an aqueous colloidal separates into two liquid phases in which one is relatively concentrated in the colloidal and the other is relatively dilute in the colloidal. When only a single type of colloidal material is present, the process is referred to as simple coacervation. However, when there is a mixture of two (oppositely charged) colloidals the process is referred to as complex coacervation. For complex coacervation to occur, the colloids should be ionized or ionisable and the conditions for coacervation require that the colloids should be oppositely charged. The conditions for coacervation may be brought about by selection of appropriately charged colloids. Alternatively, if an amphoteric colloid is employed (such as a protein, e.g. gelatine) the pH of the colloid mixture can be adjusted above or below the isoelectric point in order to impart to that amphoteric colloid the appropriate charge.

Applying complex coacervation to a process of microencapsulation, in a first step an emulsion is prepared in which tiny droplets of core-forming material are dispersed in a continuous phase consisting of a mixture of at least two-oppositely charged colloids. Coacervation is then initiated by dilution or by modifying the pH of the emulsion system or by a combination of these techniques to create phase separation. The tiny oil droplets essentially act as nucleating sites around which a liquid phase rich in colloidal material (the coacervate) will coalesce to form a liquid coating around the droplets of core material. Once the coacervate is formed around the oil droplets it is gelled. For gelling to occur, either one or both of the oppositely charged colloids must be gellable, and must be used in a concentration at which it can gel. Up until the point of gelation, the process is carried out at a temperature above the gelling point of the colloids and gelation is initiated by cooling the system to a temperature below the gelling point. In this way, soft microcapsules are formed, which will remain discrete and resist aggregation provided they are stored at a temperature below the gelling point. However, typically, the microcapsules are hardened before being further processed.

The types of colloid materials that can be employed, as stated hereinabove, must be in ionic form, or they must be ionizable. Some may be negatively charged; some may be positively charged; and some may be amphoteric, whereupon they are either positively or negatively charged depending on a material's isoelectric point and the pH of the system. Materials commonly used include animal or vegetable proteins, which include, without limitation gelatine, albumin or casein, pea protein, potato protein, whey protein, soy protein, egg protein or beta-lactoglobulin; alginates, such as sodium alginate; agar-agar; starch; pectins; carboxy-methylcellulose, gum arabic; chitosan; and other hydrophilic polymers such as copolymers of methylvinyl ether and maleic anhydride or a copolymer of polyvinylmethyl ether and maleic anhydride. Gelatine, in particular, is a very commonly used material.

When gelatine, or other protein, is employed as a microcapsule wall-forming material, it is very common to employ aldehydes as hardening agents, as they can form cross-links with amino functional groups on the protein. Typical of such aldehydes are formaldehyde and glutaraldehyde. Alternative cross-linking agents are transglutaminases, such as those disclosed in US6039901.

However, the use of either formaldehyde or glutaraldehyde is undesirable due to toxicology issues associated with both materials. Furthermore, they both cross-link by formulation of Schiff bases with amine functionality on a protein and this can lead to yellow discolouration of the microcapsules. In addition, the cross linking process using glutaraldehyde is very slow and uneconomical as a result. Cross-linking based on transglutaminase is complex, expensive and very time consuming.

There remains a need to provide new microcapsule compositions and new methods of forming microcapsule compositions that do not share the drawbacks associated with the prior art.

Applicant has surprisingly found that microcapsules consisting of droplets of core material encased in shells formed of gelled colloids comprising a protein, such as gelatine, can be hardened with polyanhydride hardening agents, to form microcapsules that are robust under conditions of handling and storage, and when in use release their core contents in a desired manner upon demand.

The invention provides in a first aspect a microcapsule composition comprising a plurality of microcapsules, a microcapsule comprising a core material encapsulated in a shell, wherein the shell comprises a complex coacervate formed from at least two oppositely charged colloids, one of which is a protein, and wherein the protein is cross linked to a hardening agent to form amide linkages between the protein and the hardening agent.

In an embodiment of the present invention the polyanhydride hardening agent contains more than one anhydride group, which is reactive with amino groups on the protein to form amide cross-linkages.

In an embodiment of the present invention the polyanhydride hardening agent comprises more than one anhydride group linked to a common central moiety. The common central moiety may be a simple organic moiety, such as an aliphatic, cycloaliphatic or aromatic moiety with a plurality of anhydride groups bonded to it. Alternatively, it may be a polymer that contains a plurality of repeat units that contain one or more anhydride groups. In either case, the anhydride groups may be bonded in a manner such that they are pendant from the common central moiety. The pendant anhydride groups may be cyclic or acyclic. Alternatively, anhydride groups may form part of the structure of the common central moiety, in which case, the anhydride groups are cyclic. Still further, the polyanhydrides may contain both pendant anhydride groups as well as anhydride groups that form a part of the structural back bone of the common central moiety.

The term "polymer" as used in relation to the common central moiety is understood to include copolymers and oligomers. Examples of suitable common central moieties include polymers of styrene, acrylic and methacrylic acids and the esters, other ethylenically unsaturated monomers and maleic anhydride, or mixtures (co-polymers) thereof.

The molecular weight of the polyanhydride may be up to about 1'000'000, and more particularly between about 300'000 and 10000 g/mol.

Suitable polyanhydride hardening agents may be selected from the group consisting of dianhydrides and other polyanhydrides. Suitable aromatic polyanhydrides include 3,3',4,4'-benzophenonetetracarboxylic dianhydride; 1,2,4,5-benzenetetracarboxylic dianhydride; 1,4,5,8-naphthalenetetracarboxylic dianhydride; and 3,4,9,10-perylenetetracarboxylic dianhydride. Suitable non-aromatic polyanhydrides include bicyclo[2,2,2]octo-7-ene-2,3,5,6-tetracarboxylic dianhydride; ethylenediaminetetraacetic dianhydride; diethylenetriaminepentaacetic dianhydride; diethylenetriaminepentaacetic dianhydride; ethylenediaminetetraacetic dianhydride; bicyclo[2.2.2]oct-7-ene-2.3.5.6-tetracarboxylic dianhydride; cyclobutane-1,2,3,4-tetracarboxylic dianhydride; 3,3',4,4' biphenyltetracarboxylic dianhydride; and perylene-3,4,9,10-tetacarboxylic dianhydride,

Suitable polymers containing anhydride functionalities include poly(ethylene-maleic anhydride); poly(maleic anhydride-1-octadecene); poly(styrene-co-maleic anhydride); poly(methyl vinyl ether-maleic anhydride); poly(ethylene-co-ethyl acrylate-co-maleic anhydride); poly(ethylene-co-vinyl acetate)-graft-maleic anhydride; polyethylene-graft-maleic anhydride; and polypropylene-graft-maleic anhydride; Poly(azelaic anhydride); poly(isobutylene-alt-maleic anhydride); poly(styrene-alt-maleic anhydride); poly(trimethylolpropane/di(propylene glycol)-alt-adipic acid/phthalic anhydride); poly[(isobutylene-alt-maleimide)-co-(isobutylene-alt-maleic anhydride)]; polystyrene-bloc-poly(ethylene-ran-butylene)-block-polystyrene-graft-maleic anhydride; and poly[(isobutylene-alt-maleic acid, ammonium salt)-co-(isobutylene-alt-maleic anhydride)] A particular example of a polyanhydride in which anhydride groups form part of the structure of the common central moiety are the ISOBAM^{®} products available from Kuraray and which consist of copolymers of isobutylene and maleic anhydride Another particular example of a polyanhydride in which anhydride groups form part of the structure of the common central moiety are the GANTREZ™ products available from Ashland and which consist of copolymers of methyl vinyl ether and maleic anhydride.

Another particular example of a polyanhydride in which anhydride groups form part of the structure of the common central moiety are the ZEMAC™ products available from Vertellus and which consist of copolymers of ethylene and maleic anhydride.

In view of the fact that the anhydride groups on the hardening agent can effectively crosslink with the amino functionality on the protein, it is possible to avoid the use of traditional aldehyde hardening agents such as formaldehyde and glutaraldehyde.

Accordingly, in another embodiment of the present invention the microcapsules are free of formaldehyde or glutaraldehyde or any other regulated mono or polyaldehyde.

In an embodiment of the present invention the microcapsule shell comprises structural units, formed by the reaction of the protein and the polyanhydride hardening agent of the following general formula 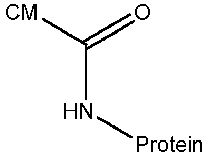 wherein
CM represents the residue of the common central moiety after the reaction of its anhydride functionality with amino functionality on a protein; and -NH-Protein is the residue of an animal or vegetable protein.

In a particular embodiment of the present invention the microcapsule shell comprises divalent structural units, formed by the reaction of the protein and the polyanhydride hardening agent, having the following general formula 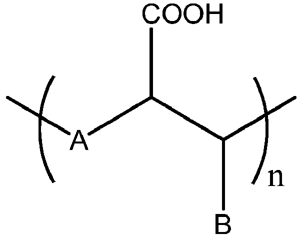 wherein A is a divalent moiety comprising 1 to 20 carbon atoms, which may be aliphatic, cycloaliphatic or aromatic; B is an acid or an amide group linked to the protein residue, more particularly an animal or vegetable protein, for example gelatine, more particularly fish gelatine; and n is an integer greater than 1.

In a more particular embodiment of the present invention the microcapsule shell comprises divalent structural units, formed by the reaction of the protein and the polyanhydride hardening agent, having the following general formula 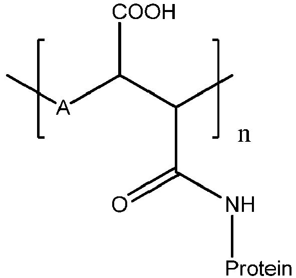 wherein A is a divalent moiety comprising 1 to 20 carbon atoms, which may be aliphatic, cycloaliphatic, or aromatic; more particularly the divalent moiety A is a group -(CRR')ₘ-, wherein R and R' are independently selected from hydrogen, methyl, or higher alkyl and m is 0, 1 or higher integer; n is an integer greater than 1 and -NH-Protein is a residue of an animal or vegetable protein, for example gelatine, more particularly fish gelatine.

The microcapsule shell-forming materials may be selected from any suitable hydrocolloid. By suitable hydrocolloid is meant a broad class of water-soluble or water-dispersible polymers that are either ionic, or are ionizable, that is, they should be anionic, cationic or zwitterionic under the particular conditions of coacervation. Hydrocolloids useful in the present invention include polycarbohydrates, such as starch, modified starch, chitosan, dextrin, maltodextrin, and cellulose derivatives, and their quaternized forms; natural gums such as alginate esters, carrageenan, xanthanes, agar-agar, pectines, pectic acid, and natural gums such as gum arabic, gum tragacanth and gum karaya, guar gums and quaternized guar gums; proteins such as gelatine, protein hydrolysates and their quaternized forms; synthetic polymers and copolymers, such as poly(vinyl pyrrolidone-co-vinyl acetate), poly(vinyl alcohol-co-vinyl acetate), poly((met)acrylic acid), poly(maleic acid), poly(alkyl(meth)acrylate-co-(meth)acrylic acid), poly(acrylic acid-co-maleic acid)copolymer, poly(alkyleneoxide), poly(vinylmethylether), poly(vinylether-co-maleic anhydride), and the like, as well as poly-(ethyleneimine), poly((meth)acrylamide), poly(alkyleneoxide-co-dimethylsiloxane), poly(amino dimethylsiloxane), and the like, and their quartenized forms.

The core material may be selected from a wide variety of materials in which one would want to deliver from a consumer product. Non-limiting examples of active materials include pharmaceuticals, drugs, pro-drugs, neutraceuticals, flavouring agents, perfumes, fungicide, brighteners, antistatic agents, anti-bacterials, wrinkle control agents, fabric softener actives, hard surface cleaning actives, UV protection agents, insect repellants, animal/vermin repellants, flame retardants, conditioning agents, dyes, coolants and the like.

In a particular embodiment, the core material comprises a fragrance, in which case the microcapsules containing said fragrance provide a controlled-release of fragrance into an environment to be perfumed. In this case, the fragrance is typically comprised of a number of fragrance ingredients, which may include essential oils, botanical extracts, synthetic perfume materials, and the like. A list of suitable fragrance ingredients can be found in specialized books of perfumery, e.g. in S. Arctander (Perfume and Flavor Chemicals, Montclair N.J., USA 1969 or later versions thereof), or similar textbooks of reference.

In general, the core material is contained in the microcapsule at a level of from about 1% to about 99 %, preferably from about 10 % to about 97 %, and more preferably from about 30 % to about 95 %, by weight of the total microcapsule. The weight of the total microcapsule includes the weight of the shell of the microcapsule plus the weight of the core material inside the microcapsule.

The microcapsules of the present invention are distinguished by several advantages that derive from the use of a hardening agent containing reactive anhydride functionality: The hardening process proceeds rapidly, that is, in the order of 1 to 3 hours, compared with a process employing glutaraldehyde or formaldehyde, which can take upwards of 6 hours. Still further, because anhydrides are employed, the cross-linkages formed are amide groups; whereas formaldehyde and glutaraldehyde forms Schiff base cross-linkages that are associated with yellow discolouration. By contrast, microcapsule compositions of the present invention are water white and do not undergo any discoloration over time whatever the pH of use.

A particular advantage of the microcapsule composition of the present invention is their ability to uptake high loadings of fragrance ingredients and to retain them in the core without losing substantial amounts back into the environment through leakage. Without wishing to be bound by any particular theory, the applicant believes that not only is the anhydride a surprisingly efficient cross-linking agent, but carboxylic acid groups that form as a result of the cross linking reaction can form hydrogen bonds with functional groups contained on the protein or other shell-forming materials. This combination of cross-linkages and hydrogen bonding interactions can lead to particularly impermeable microcapsule shells.

Furthermore, whereas crosslinking based on Schiff Bases can be prone to degradation by hydrolysis even under slightly acidic or basic conditions, microcapsule compositions of the present invention are stable within a pH range of about 3 to about 10, more particularly about 3 to about 8.

Fragrance ingredients can be introduced into the microcapsules during microcapsule formation. However, this is a particularly complicated task to undertake when the microcapsule is formed by a process of coacervation. As stated above, fragrance compositions typically consist of many fragrance ingredients with disparate molecular weights, volatilities, partition coefficients or solubilities in water. This will mean that each ingredient will possess a unique propensity for being retained in the core material or for being dispersed into the aqueous discontinuous phase. Fragrance ingredients with a propensity to partition into an aqueous phase can be particularly difficult to encapsulate because of their propensity not to remain static within the core and because they can affect the stability of the colloid.

For this reason, instead of attempting to encapsulate fragrance compositions during the formation of microcapsules by coacervation, it is conventional to post-load fragrance compositions into microcapsules once they have been formed. The technique of post-loading microcapsules formed by coacervation is known in the art (see for example WO9917871). In a typical process, a core-shell capsule is formed by coacervation. The shell typically consists of a protein, such as gelatine, a carbohydrate, and optionally other synthetic film-forming polymers. At this time, the microcapsules contain a blank core, that is to say, the core material consists only of an oil, such as a vegetable oil, mineral oil, benzyl alcohol, or a mixture thereof. The fragrance composition is introduced into the capsule core by mixing it in water and adding the mixture to the dried microcapsules. The capsule shell swells as it is hydrated, and the fragrance composition passes through the swollen shell by a process of diffusion. The uptake of the fragrance composition into the microcapsule cores will depend upon the amount of water that is able to penetrate the microcapsule shells. If the amount of water is relatively low, the solubility of the fragrance ingredients in the water will be concomitantly low and the partitioning of fragrance into the oil cores is promoted. If, on the other hand, the amount of water in the shells is high, the converse is true, and the tendency for the fragrance composition to diffuse into the core will be reduced.

A problem with gelatine coacervate microcapsules that have been hardened using formaldehyde or glutaraldehyde or glyoxal is that the shells can be very permeable. When subjected to post-loading treatment, the microcapsules swell a great deal, the uptake of water is high, and as a result the amount of fragrance entering the core and being retained in the microcapsule can be quite low.

Microcapsules of the present invention exhibit greater impermeability compared with coacervate capsules hardened with formaldehyde or glutaraldehyde. The concentration of water entering the shells is quite low during post-loading, partitioning of fragrance ingredients into the cores is promoted, and as such, fragrance loading and retention in the cores is high.

In another aspect of the present invention there is provided a method of forming a microcapsule composition as herein above defined.

The process of forming the microcapsule composition of the present invention may be characterised by the following steps:
I) forming a dispersion of tiny droplets of core material in a coacervate of oppositely charge colloids, one of which colloids is a protein, and forming a coating of coacervate around the droplets
II) gelling the coacervate coating by reducing the temperature below the gel temperature of the coacervate to formed gelled microcapsules; and
III) hardening the gelled microcapsules by the addition of a hardening agent that reacts with the protein to form amide cross-linkages. The coating step I) can be achieved by first forming an oil-in-water emulsion comprising a dispersion of tiny droplets of core material in an aqueous mixture of at least two oppositely charged colloids, one of which is a protein, before applying a phase-inducing agent to the emulsion to cause the colloids to coacervate and condense around the droplets to form a liquid coacervate coating around said droplets.

Alternatively, an aqueous mixture of at least two oppositely charged colloids, one of which is a protein, is caused to form a coacervate by applying a phase-inducing agent to the aqueous mixture, before adding the core material to the coacervate, dispersing the core material as tiny droplets in the coacervate, and allowing the coacervate to condense around the tiny droplets to form a liquid coacervate coating around the droplets.

Process steps I) and II) are conventional in the art of forming microcapsules by a process of complex coacervation.

In a particular embodiment of the invention, in step I) the oil-in-water emulsion is formed when the aqueous mixture of the colloids is mixed energetically with an oil phase of core-forming material. The colloids are either anionic, cationic or amphoteric. A list of suitable colloids is set forth above. At least one of the materials is a protein, such as gelatine. Proteins are amphoteric and may be positively or negatively charged depending on the pH of the system. Then at least one additional colloids should bear the opposite charge to the protein under the conditions of coacervation. At least one of the colloids should be capable of forming a gel, and the gellable material(s) should be employed at a concentration such that it is able to form a gel around the droplets of core material. A typical concentration of gellable colloid in the aqueous continuous phase is about 0.5 % or more, more particularly about 0.5 % to 50 %.

The term "phase-inducing agent" used herein above means any agent(s) or any process condition(s), which when introduced or applied to the colloid will cause the formation of a coacervate. Phase-inducing agents are well known in the art, and coacervation is typically initiated with the introduction of water or other water-miscible solvent, such as methanol or ethanol, in a dilution step, or a change of pH or a change of temperature, or by a combination of these measures, as is generally well known in the art. If pH adjustments are necessary, for example, to raise it or lower the pH above or below the isoelectric point of the particular protein employed, then this may be carried out using an acid or a based as appropriate.

Prior to the gelling step, the process is carried out above the gelling temperature of the coacervate formed around the droplets of core material. Gelling of the coacervate formed around the droplets of core material is effected by reducing the temperature of the system below the gelling point of the coacervate. After gelling, the resultant slurry of soft microcapsules is generally sufficiently durable and will remain in unaggregated form provided the slurry is agitated with stirring. However, in order to produce robust microcapsules that are capable of withstanding further processing, it is necessary to subject the soft microparticles to a hardening step.

As stated hereinabove, traditionally, microcapsules formed by a process of coacervation of a protein-containing mixture of colloids are usually hardened using an aldehyde such as formaldehyde, glutaraldehyde or glyoxal

The process of the present invention is distinguished over the prior art in that microcapsules formed are hardened using polymeric materials containing anhydride functionality, rather than the commonly employed aldehydes described above. As the cross-linking reaction depends upon the reaction of an anhydride with amine groups on the protein, the pH of the reaction medium is buffered to a neutral or weakly basic pH to ensure that the amino groups are not quaternized. A suitable pH for the hardening step is in the range of about 9 to about 11. The temperature may be held below the gelling temperature of the gellable colloids, which in the case of gelatine is typically in the range of about 5 to 30 degrees centigrade during the hardening step.

In a process according to the invention, the ratio (weight/weight) of protein to polyanhydride should be between 1 : 0.01 to 1 : 10, more particularly 1 : 0.01 to 1 : 1, still more particularly 1 : 0.25.

In the hardening step, the hardening agent is added to a slurry containing the soft microcapsules. The hardening agent may be added to the capsule slurry in the form of a solution or suspension in a convenient vehicle such as water or a water-miscible solvent or mixture thereof, or it may be added in pulverulent form. However, in order to minimize the risk of any undesirable hydrolysis and deactivation of the anhydride functionality in the hardening agent, it is preferred that it is added to the slurry in pulverulent form.

The use of a hardening agent containing anhydride functionality in the formation of microcapsules is new and counter-intuitive. The skilled person would consider that in an aqueous environment, the kinetics of the hydrolysis and ring-opening of the polyanhydride would be highly competitive with the kinetics of amide formation by means of the reaction of an anhydride with an amine. However, surprisingly the applicant has found that when the polyanhydride is added to the slurry of soft microcapsules, they react readily with the proteins in the shell wall to form amide cross-linkages.

In the preparation of microcapsules by coacervation, it is known to use solutions of certain wall-forming anhydride co-polymers, such as polyvinylmethylether-maleic anhydride or polyethylenemaleic anhydride copolymer (see for example US 3,533,958 and US 3,041,289). Specifically, aqueous solutions of these film-forming copolymers are used in combination with gelatine and carboxymethyl cellulose colloids to form coacervate shells around oil cores. However, the use of these film-forming copolymers is distinct from and is not to be confused with, the use of anhydrides as hardening agents as set forth in the present invention. In the case of the prior art use of anhydride copolymers, the copolymers are used in aqueous solution. Under such conditions the anhydride functionality will be hydrolysed to its corresponding acid form or its salt. The kinetics of amide formation by the reaction of a carboxylic acid and an amine are not favourable. Indeed, amide formation will not occur without special reaction conditions or catalysis. Without special precautions, carboxylic acids will merely protonate amino functionality to form a quaternary salt of the amine. Accordingly, the manner in which these prior art anhydride copolymers are employed, they are not functioning, and cannot function, as hardening agents.

The process according to the present invention results in the formation of a microcapsule composition comprising a plurality of microcapsules suspended as a slurry in an aqueous carrier. If the microcapsule composition is stored and further processed in the form of a slurry, the pH of the slurry is adjusted to about 3 to 8 by the addition of a suitable acid, such as citric acid or formic acid and a preservative added.

If desired the microcapsule composition may be dried and stored in pulverulent form. Drying may be carried out directly by spray drying or by fluid bed drying. Alternatively, the microcapsule composition can be dried by decanting off the liquid from the slurry and drying the microcapsules in an oven to produce a cake, which can then be rendered in pulverulent form by a subsequent comminution step. However the microcapsule composition is dried, in order to prevent aggregation and improve the bulk flow properties of the microcapsules it may be desirable to add a flow aid to the microcapsule composition before the drying process. Suitable flow aids will be known to the skilled person in the art and will include, without limitation silica, starch, calcium carbonate and sodium sulphate.

The size of the microcapsules can be important in the usefulness of microcapsule compositions according to the practice of the present invention. Capsules can be prepared having a mean diameter of from about 0.001 to about 1,000 microns, preferably from about 1 to about 500 microns, more preferably from about 10 to about 100 microns, and even more preferably from about 10 to about 70 microns. These dimensions can play an important role in the ability to control the application of the microcapsule composition in the practice of the present invention. The broadest range of microcapsule size under any conditions would be about 0.001 to about 1,000 microns and a more easily sprayed size limit would be between about 20 and about 85 microns.

The mean particle size can be determined in a manner known in the art. A particular method of measuring particle size is light scattering. Light scattering measurements can be made using a Malvern Mastersizer.

Applicant has found, in accordance with the present invention, that capsules having a mean particle size (D50) of 10 to250 microns are more resistant to leakage, have good odourant oil retention, and are particularly mechanically resistant, which makes them particularly suitable for applications in which activated release is important.

Increasingly, commercial interest resides in capsules that provide relatively little perfume impression before they are activated by some external stimulus, for example, mechanical agitation. Pre-activation hedonic contribution can be provided by free (i.e. non-encapsulated) perfume oil, such that the total olfactive performance of a perfume system is made up of both encapsulated and non-encapsulated fragrance oil. As such, a capsule's mechanical performance must be balanced between a relatively small population of capsules that are broken in an early phase of consumer usage, and a relatively larger population of capsules that are resistant to breakage and are still intact towards the end of consumer usage, such that the consumer receives a continuous signal of a product's efficacy throughout the duration of consumer usage. Capsules exhibiting the aforementioned mean particle size (D 50) provide a particularly balanced olfactive profile.

When the microcapsule composition is in the form of a suspension in an aqueous carrier it is desirable to employ a stabilising agent in the microcapsule core to prevent or reduce the amount of fragrance composition that may leach out of the microcapsules. Stabilizing agents include isopropyl myristate, triethyl citrate, mineral oil, silicone oil, diethyl propyl acteate, benzyl phenyl acetate, citronellyl phenyl acetate, benzyl isoeugenol, diphenyl oxide, gamma-dodecalactone, dibutyl phthalate, methyl myristate, ethyl myristate, ethyl palmitate, benzyl salicylate, benzyl benzoate, phenyl ethyl phenyl acetate, geranyl phenyl aceate, benzyl cinnamate, ethylene brassylate, ambretone, galaxolide, tonalid, exaltolide, habanolide, iso-amyl laurate, cedryl acetate, hexyl cinnamic aldehyde, patchouli alcohol, delta-guaiene, delta-cadinene, alcohols of C10 or greater, Dowanolg, dipropylene myristate and tripropylene myristate, Isopar(R) orange terpenes, and mixtures thereof.

It is desirable for a suspension of microcapsules to contain a dispersant. Dispersants are employed to ensure that the microcapsules remain suspended and tend not to cream or sediment. Suitable dispersants include pectine, alginate, arabinogalactan, carageenan, gellan gum, xanthum gum, guar gum, acrylates/acrylic polymers, water-swellable clays, fumed silicas, acrylate/aminoacrylate copolymers, and mixtures thereof. Preferred dispersants herein include those selected from the group consisting of acrylate/acrylic polymers, gellan gum, fumed silicas, acrylate/aminoacrylate copolymers, water-swellable clays, and mixtures thereof.

In order to prevent microbial contamination it is desirable that the microcapsule composition contains a preservative. The preservative may be contained in the core material and/or in the aqueous carrier. Suitable preservatives include quaternary compounds, biguanide compounds, and mixtures thereof. Non-limiting examples of quaternary compounds include benzalkonium chlorides and/or substituted benzalkonium chlorides such as commercially available Barquat(R) (available from Lonza), Maquat(R) (available from Mason), Variquat(R) (available from Witco/Sherex), and Hyamine(R) (available from Lonza); di(C6-C14)alkyl di short chain (C1-4 alkyl and/or hydroxyalkl) quaternary such as Bardac(R) products of Lonza; N-(3-chloroallyl) hexaminium chlorides such as Dowicide(R) and Dowicil(R) available from Dow; benzethonium chloride such as Hyamine(R) from Rohm & Haas; methylbenzethonium chloride represented by Hyamine(R) 10* supplied by Rohm & Haas, cetylpyridinium chloride such as Cepacol chloride available from of Merrell Labs; and diester quaternary ammonium compounds. Examples of preferred dialkyl quaternary compounds are di(C8-C12)dialkyl dimethyl ammonium chloride, such as didecyldimethylammonium chloride (Bardac(R) 22), and dioctyldimethylammonium chloride (Bardac(R) 2050). The quaternary compounds useful as cationic preservatives and/or antimicrobial agents herein are preferably selected from the group consisting of dialkyldimethylammonium chlorides, alkyldimethylbenzylammonium chlorides, dialkylmethylbenzylammonium chlorides, and mixtures thereof. Other preferred cationic antimicrobial actives useful herein include diisobutylphenoxyethoxyethyl dimethylbenzylammonium chloride (commercially available under the trade name Hyamine(R) 1622 from Rohm & Haas) and (methyl)diisobutylphenoxyethoxyethyl dimethylbenzylammonium chloride (i.e. methylbenzethonium chloride).

Non-limiting examples of biguanide compounds include 1,1'-hexamethylene bis(5-(p-chlorophenyl)biguanide), commonly known as chlorhexidine, and Cosmoci(R) CQ(R), Vantocil(R) IB, including poly (hexamethylene biguanide) hydrochloride. Other useful antimicrobial actives include the bis-biguanide alkanes. Usable water soluble salts of the above are chlorides, bromides, sulfates, alkyl sulfonates such as methyl sulfonate and ethyl sulfonate, phenylsulfonates such as p-methylphenyl sulfonates, nitrates, acetates, gluconates, and the like.

Non-limiting examples of other suitable antimicrobial actives include Pyrithiones (especially the zinc complex (ZPT)), Octopirox(R), Dimethyldimethylol Hydantoin (Glydant(R)), Sodium Sulfite, Sodium Bisulfite, Imidazolidinyl Urea (Germall 115(R)), Diazolidinyl Urea (Germall II(R), Benzyl Alcohol, 2-Bromo-2-nitropropane-1,3-diol (Bronopol(R)), Formalin (formaldehyde), lodopropenyl Butylcarbamate (Polyphase P100(R)), Chloroacetamide, Methanamine, Methyldibromonitrile Glutaronitrile (1,2-Dibromo-2,4-dicyanobutane or Tektamer(R)), Glutaraldehyde, 5-bromo-5-nitro-1,3-dioxane (Bronidox(R)), Phenethyl Alcohol, o-Phenylphenol/sodium o-phenylphenol, Sodium Hydroxymethylglycinate (Suttocide A(R)), Polymethoxy Bicyclic Oxazolidine (Nuosept C(R)), Dimethoxane, Thimersal, Dichlorobenzyl Alcohol, Captan, Chlorphenenesin, Dichlorophene, Chlorbutanol, Glyceryl Laurate, Halogenated Diphenyl Ethers, 2,4,4'-trichloro-2'-hydroxy-diphenyl ether (Triclosan(R) or TCS), 2,2'-dihydroxy-5,5'-dibromo-diphenyl ether, Phenolic Compounds (as described in U.S. Pat. No. 6,190,674), Para-chloro-meta-xylenol (PCMX), Chlorothymol, Phenoxyethanol, Phenoxyisopropanol, 5-Chloro-2-hydroxydiphenylmethane, Resorcinol and its Derivatives (as described in U.S. Pat. No. 6,190,674), 5-Chloro 2,4-Dihydroxydiphenyl Methane, 4'-Chloro 2,4-Dihydroxydiphenyl Methane, 5-Bromo 2,4-Dihydroxydiphenyl Methane, 4'-Bromo 2,4-Dihydroxydiphenyl Methane, Bisphenolic Compounds, Parabens, Halogenated Carbanilides, and mixtures thereof.

In addition to any fragrance formulation that may be contained within the microcapsules, a slurry of microcapsules of the present invention may also contain free perfume in the suspending medium.

The microcapsule composition of the present invention can be employed for a large number of purposes and for delivery a wide variety of active agents. Preferably, however, the microcapsule compositions are used as delivery vehicles for flavour or fragrance formulations.

In general, the microcapsule composition of the present invention may be used in consumer products in a wide variety of levels. Microcapsule compositions are typically employed consumer products such that the amount of microcapsules represents about 0.001% to about 99.9% by weight of the total weight of the consumer product, preferably from about 0.005% to about 50%, and more preferably from about 0.01% to about 20%, by weight of the consumer product.

A microcapsule composition of the present invention may be added to consumer products in the form of a dry powder or as a slurry in suitable carrier liquid, in particular an aqueous carrier.

All manner of consumer products, such as fine fragrances or personal care or household care products may contain the microcapsule composition of the present invention.

The microcapsule composition according to the invention can thus constitute a composition for scenting, caring for or treating consumer products and can in particular be provided in the form of eau fraiche, eau de toilette, eau de parfum, aftershave lotion, care water, silicon or aqueous/silicone care oil or anhydrous cream. It can also be provided in the form of a scented two-phase lotion (eau de toilette phase/hydrocarbon oil and/or silicon oil phase).

A microcapsule composition according to the present invention can be provided in all manner of physical forms, and in particular in the form of aqueous gels or of aqueous or aqueous/alcoholic solutions. They can also, by addition of a fatty or oily phase, be provided in the form of dispersions of the lotion type, of emulsions with a liquid or semiliquid consistency of the milk type, obtained by dispersion of a fatty phase in an aqueous phase (O/W) or vice versa (W/O), or of suspensions or emulsions with a soft, semisolid or solid consistency of the cream or gel type, or also of multiple (W/O/W or O/W/O) emulsions, of micro-emulsions, of vesicular dispersions of ionic and/or nonionic type, or of wax/aqueous phase dispersions.

There now follows a series of examples that serve to illustrate the invention.

### Example 1

### Preparation of capsules with Poly(methyl vinyl ether co maleic anhydride)

Capsules are prepared by pre-warming deionized water to 50°C. A gum solution is prepared by vigorously agitating pre-warmed deionized water (77.99g), carboxymethyl cellulose, sodium salt (1.65g). The solution is mixed until the solids are completely dissolved, then the solution is cooled to about 35°C to about 40°C. A gelatin solution is prepared by vigorously agitating pre-warmed deionized water (145.82g) and 250 Bloom fish gelatin (16.5g) in a pre-emulsion tank until the gelatin is completely dissolved, then the solution is cooled to about 35°C to about 40°C. Without agitation, the gum solution is added to the gelatin solution in the pre-emulsion tank. The pH is adjusted to about 7 with either a dilute sodium hydroxide solution (50% w/w) or a dilute citric acid solution (50% w/w).

Vegetable oil (Miglyol) or fragrance (164.75g) is added with slow agitation. The capsule size is adjusted to about 20 to 400 microns and the size is verified microscopically. Once capsule size is reached, pre-warmed deionized water (475.67g) is added. The pH is adjusted to about 5.5 with either a dilute sodium hydroxide solution (50% w/w) or a dilute citric acid solution (50% w/w).The solution is slowly cooled at about 1°C per 5 min until the solution reaches about 28°C.

If the capsule walls are intact, as determined by microscopic examination of capsules showing uniform deposition of protein with no free protein floating in the water phase, the solution may be quickly cooled to about 10°C. If the capsule walls are thin, as determined by microscopic examination of capsules showing non-uniform deposition of protein and free protein floating in the water phase, the solution is reheated to about 32°C to about 33°C. The solution is mixed at about 5°C to about 10°C for 1h. The solution is then heated to about 15°C to about 20°C.

The pH is adjusted to about 10 with a dilute sodium hydroxide solution (50% w/w). Poly(methyl vinyl ether co maleic anhydride) of a Mw of 216000 g/mol (2g) in powder under vigorous agitation. Re-adjust pH to about 10 with a dilute sodium hydroxide solution (50% w/w) every 15 minutes until pH stabilization to 10. This step takes about 2 hours.

Sodium benzoate (10% w/w) is added with thorough mixing and pH is adjusted to less than 4 with citric acid.

Microcapsules shell is still visible if capsules are heated in water above the gelling point of gelatin up to 100°C.

When fragrance is encapsulated and microcapsules dried, solid content measured is conform to theoretical solid content (18.3%). Fragrance is kept inside the microcapsules upon drying showing the effectiveness of the cross-linking step.

A first batch of capsules made with this process and fragrance showed a particle size of 25 microns and a solid content of 19.5 %. Another batch of capsules made with this process and a fragrance showed a particle size of 60 microns and a solid content of 20%.

### Example 2

### Preparation of capsules with poly(ethylene-alt-maleic) anhydride

Capsules are prepared by pre-warming deionized water to 50°C. A gum solution is prepared by vigorously agitating pre-warmed deionized water (77.99g), carboxymethyl cellulose, sodium salt (1.65g). The solution is mixed until the solids are completely dissolved, then the solution is cooled to about 35°C to about 40°C. A gelatin solution is prepared by vigorously agitating pre-warmed deionized water (145.82g) and 250 Bloom type A Fish gelatin (16.5g) in a pre-emulsion tank until the gelatin is completely dissolved, then the solution is cooled to about 35°C to about 40°C. Without agitation, the gum solution is added to the gelatin solution in the pre-emulsion tank. The pH is adjusted to about 7 with either a dilute sodium hydroxide solution (50% w/w) or a dilute citric acid solution (50% w/w).

Fragrance (164.75g) is added with slow agitation. The capsule size is adjusted to about 20 to 400 microns and the size is verified microscopically. Once capsule size is reached, pre-warmed deionized water (475.67g) is added. The pH is adjusted to about 5.5 with either a dilute sodium hydroxide solution (50% w/w) or a dilute citric acid solution (50% w/w).The solution is slowly cooled at about 1°C per 5 min until the solution reaches about 28°C.

If the capsule walls are intact, as determined by microscopic examination of capsules showing uniform deposition of protein with no free protein floating in the water phase, the solution may be quickly cooled to about 10°C. If the capsule walls are thin, as determined by microscopic examination of capsules showing non-uniform deposition of protein and free protein floating in the water phase, the solution is reheated to about 32°C to about 33°C. The solution is mixed at about 5°C to about 10°C for 1h. The solution is then heated to about 15°C to about 20°C.

The pH is adjusted to about 10 with a dilute sodium hydroxide solution (50% w/w). ZEMAC E60, Molecular weight of 60000 g/mol (2g) in powder under vigorous agitation. Re-adjust pH to about 10 with a dilute sodium hydroxide solution (50% w/w) every 15 minutes until pH stabilization to 10. This step takes about 2 hours.

Sodium benzoate (10% w/w) is added with thorough mixing and pH is adjusted to less than 4 with citric acid.

Microcapsules shell is still visible if capsules are heated in water above the gelling point of gelatin up to 100°C.

Solid content measured conforms to a theoretical solid content (19.7%). This second batch showed a particle size of 15 microns. Fragrance is kept inside the microcapsules upon drying showing the effectiveness of the cross-linking step.

### Example 3

### Preparation of capsules with poly(ethylene-alt-maleic) anhydride - ZEMAC E400 -High molecular weight.

Capsules are prepared by pre-warming deionized water to 50°C. A gum solution is prepared by vigorously agitating pre-warmed deionized water (77.99g), carboxymethyl cellulose, sodium salt (1.65g). The solution is mixed until the solids are completely dissolved, then the solution is cooled to about 35°C to about 40°C. A gelatin solution is prepared by vigorously agitating pre-warmed deionized water (145.82g) and 250 Bloom type A Fish gelatin (16.5g) in a pre-emulsion tank until the gelatin is completely dissolved, then the solution is cooled to about 35°C to about 40°C. Without agitation, the gum solution is added to the gelatin solution in the pre-emulsion tank. The pH is adjusted to about 7 with either a dilute sodium hydroxide solution (50% w/w) or a dilute citric acid solution (50% w/w).

Miglyol (164.75g) is added with slow agitation. The capsule size is adjusted to about 20 to 400 microns and the size is verified microscopically. Once capsule size is reached, pre-warmed deionized water (475.67g) is added. The pH is adjusted to about 5.5 with either a dilute sodium hydroxide solution (50% w/w) or a dilute citric acid solution (50% w/w).The solution is slowly cooled at about 1°C per 5 min until the solution reaches about 28°C.

If the capsule walls are intact, as determined by microscopic examination of capsules showing uniform deposition of protein with no free protein floating in the water phase, the solution may be quickly cooled to about 10°C. If the capsule walls are thin, as determined by microscopic examination of capsules showing non-uniform deposition of protein and free protein floating in the water phase, the solution is reheated to about 32°C to about 33°C. The solution is mixed at about 5°C to about 10°C for 1h. The solution is then heated to about 15°C to about 20°C.

The pH is adjusted to about 10 with a dilute sodium hydroxide solution (50% w/w). ZEMAC E400 (2g) in powder under vigorous agitation. Re-adjust pH to about 10 with a dilute sodium hydroxide solution (50% w/w) every 15 minutes until pH stabilization to 10. After 3 hours, pH was still moving indicating the reaction was not finished.

Sodium benzoate (10% w/w) is added with thorough mixing and pH is adjusted to less than 4 with citric acid.

Microcapsules shell is still visible if capsules are heated in water above the gelling point of gelatin up to 100°C but looked softer than example with ZEMAC E60.

The particle size is 45 microns.

This example shows that ZEMAC E400 is less reactive towards gelatin than ZEMAC E60.

### Example 4

### Synthesis of fluorescently labelled gelatin coacervate particles, crosslinked with polyanhydride ), formed according to the process of Example 1 (designated P-1) and particles crosslinked with glutaraldehyde (designated P-A); and Synthesis of shampoo comprising P-1 (in accordance with the invention) or P-A (comparative)

Fluorescently labelled gelatin coacervate particles were prepared containing 0.02% Solvent Yellow 98 dye (Hostasol Yellow 3G™ ex. Clariant) dissolved in Miglyol^{®} triglyceride oil.

Fluorescently labelled glutaraldehyde cross-linked gelatin complex coacervate particles (designated P-A) were prepared as a dry powder.

Fluorescently labelled poly(anhydride) cross-linked gelatin complex coacervate particles (designated P-1) were prepared as a 22.5% solids aqueous slurry.

A model shampoo formulation was prepared leaving a "hole" for post-dosage of other components by omitting 10% w/w of the water. Working slurries containing 20% solids of P-A and P-1 capsules in water were also generated by dilution in high purity (Milli-Q™) water.

Shampoo formulations (designated SP-1 and SP-A)were prepared by post-dosing P-1 and P-A capsules respectively and adjusting the water content. The final capsule inclusion level was 1% w/w solids. The compositions were gently mixed for 8 hours and then stored at ambient temperature.

A capsule free Control formulation was prepared by addition of water only.

The compositions of the formulations are listed in Table 1.

**Table 1: Composition of model shampoo base SP-1 (in accordance with the invention) and comparative shampoo base SP-A.**

| | **% inclusion (w/w)** | | |
|---|---|---|---|
| **Ingredient** | **SP-A** | **SP-1** | **Shampoo** |
| Anionic surfactant | 19.0 | 19.0 | 19.0 |
| Cationic surfactant | 5.3 | 5.3 | 5.3 |
| Thickener | 0.4 | 0.4 | 0.4 |
| Silicone oils | 4.3 | 4.3 | 4.3 |
| Cationic polymer | 0.20 | 0.20 | 0.20 |
| Sequestrant | 0.25 | 0.25 | 0.25 |
| pH buffer | To pH 5.5-6 | To pH 5.5-6 | To pH 5.5-6 |
| Capsule P-A | 1.0 | 0 | 0 |
| Capsule P-1 | 0 | 1.0 | 0 |
| Water and minors (including preservatives) | to 100 | to 100 | to 100 |

### Example 5

### Fluorescer leakage from capsules into a model shampoo

After a designated storage period, 0.5 g of shampoo formulation was removed from the sample and mixed with 49.5 g of high purity (Milli-Q™) water to produce a 1 in 100 diluted sample. The solution was centrifuged at 10,000 rpm for 15 minutes to separate out solids before the liquid supernatant was passed through a 1.2 micron glass microfibre syringe filter directly into a 1 cm pathlength cuvette.

The extent of fluorescer leakage from the capsules was assessed by measuring the fluorescence spectrum at an excitation wavelength of 460 nm over an emission wavelength range of 480 to 600 nm. The emission maximum was found to be 503 nm. Three replicate measurements were made at each time point for each formulation.

A series of fluorescer standards were prepared by addition of aliquots of Hostasol Yellow 3G™ dye in acetone to 1 in 100 diluted Control shampoo. These solutions were centrifuged and syringe filtered in the manner described above.

The extent of dye leakage from the capsules into the shampoo base was calculated by comparison with the calibration curve.

The results are tabulated in Table 2.

**Table 2: Mean amount (%) of fluorescer leakage into shampoo base at ambient temperature for SP-1 and SP-A**

| **Storage time** | **SP-A** | **SP-1** |
|---|---|---|
| 8 hours | 16.8 | 0.9 |
| 12 days | 85.9 | 35.5 |
| 26 days | 91.6 | 77.5 |

The results clearly show that leakage of the hydrophobic fluorescer is significantly more rapid in SP-A, containing the glutaraldehyde cross-linked capsules, compared to the polymeric anhydride cross-linked capsules used in SP-1.

### Example 6

### Fluorescer leakage from capsules into a model roll-on deodorant formulation

20% solids suspension of P-A and P-1 capsules in high purity water were prepared.

A model deodorant roll-on base was added to the capsule slurries to prepare formulations DP-1 and DP-A. The capsule inclusion level was 1% w/w solids. A capsule free Control formulation was prepared by the same volume of high purity water. The compositions were gently mixed for 8 hours and then stored at ambient temperature.

The compositions of the formulations are listed in Table 3.

**Table 3: Composition of model deodorant base DP-1, in accordance with the invention and comparative deodorant base DP-A.**

| | **% inclusion (w/w)** | | |
|---|---|---|---|
| **Ingredient** | **DP-A** | **DP-1** | **Control** |
| Sunflower oil | 1.9 | 1.9 | 1.9 |
| Steareth-2 | 2.47 | 2.47 | 2.47 |
| Steareth-20 | 0.57 | 0.57 | 0.57 |
| Perfume | 0.57 | 0.57 | 0.57 |
| Capsule P-A | 1.0 | 0 | 0 |
| Capsule P-1 | 0 | 1.0 | 0 |
| Water | to 100 | to 100 | to 100 |

After a designated storage period, 0.5 g of deodorant roll-on formulation was removed from the sample and mixed with 49.5 g of high purity water to give a 1 in 100 diluted sample. The solution was centrifuged and filtered as described in Example 1. The degree of fluorescer leakage was assessed by comparison with fluorescer standards prepared by addition of aliquots of Hostasol Yellow 3G™ dye dissolved in acetone to 1 in 100 diluted Control roll-on, which were centrifuged and filtered in the same manner. The emission maximum was found to be 500 nm and three replicate measurements were made at each time point for each formulation.

The results are tabulated in Table 4 below.

**Table 4: Mean amount (%) of fluorescer leakage into deodorant base at ambient temperature for DP-1 and DP-A**

| **Storage time** | **DP-A** | **DP-1** |
|---|---|---|
| 8 hours | 18.8 | 3.7 |
| 1 days | 29.2 | 5.1 |
| 4 days | 31.5 | 9.7 |
| 13 days | 32.4 | 10.6 |
| 23 days | 34.2 | 12.5 |

It will be seen that leakage of the hydrophobic fluorescer is significantly more rapid in DP-A, containing the glutaraldehyde cross-linked capsules, compared to the polymeric anhydride capsules used in DP-1.

### Example 7

An experimental perfume A ("woody-floral" ) was encapsulated using the encapsulation method set out in Example 1 above. The composition of the perfume "woody-floral" is set out in the table, below. In addition, the perfume contained 0.02% fluorescein dye for better microscopic examination.

| Perfume Ingredient | % |
|---|---|
| Ionone-beta | 20 |
| Kohinool | 20 |
| Hedione | 20 |
| Javanol | 20 |
| Lilial | 20 |

A gelatin capsule using glutaraldehyde as a crosslinker (Gelatin benchmark) was used to illustrate improvement of performance with the gelatin capsules of the present invention.

The mechanical stress properties of the gelatine capsules according to the invention were investigated and compared to the gelatine benchmark. The method used for this assessment is based on a simple "rolling pin test". Capsules were diluted in water at 10% and 10µg of the dilution containing the said capsules was applied on a microscopic glass slide and allowed to dry for 2 hours.

The slides were then examined under a light microscope upon submitting both capsules to the rolling-pin test.

A glass slide was put on top of the one containing the sample. A cylindrical object (360g) was rolled on top of the glass slides and the sample was then evaluated by light microscope under fluorescent light. This simple test provided an easy method to apply a constant vertical force (360 grams) on all samples tested.

The above microscope examination was then repeated after 5 pin rolls and finally 10 pin rolls. Finally a last step consists of turning the top microscope slide at 90 degree angle and sliding it on the entire bottom slide to add an extra shear step to the samples containing the capsules.

The percentage of broken capsules was quantified based on eye examination, in a straightforward manner, as the lack of structural integrity in addition to fluorescent oil was clearly evident in the continuous phase when the capsules are broken.

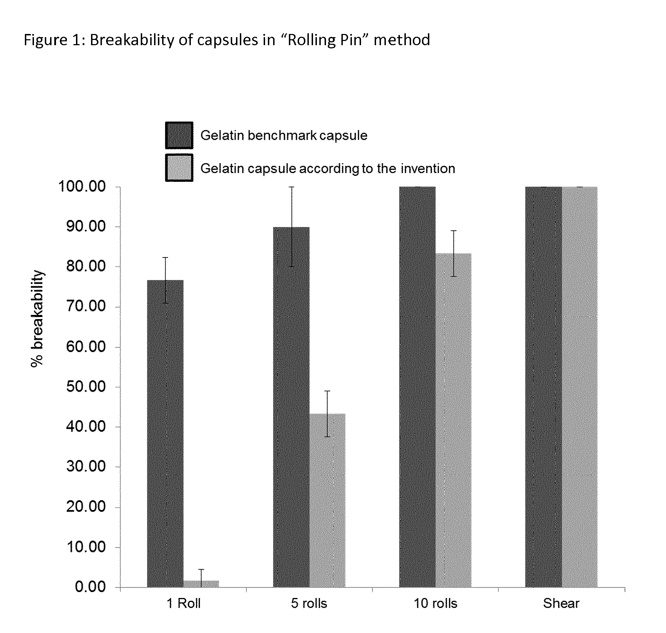

The results are shown graphically in Figure 1:

As illustrated in Figure 1, the gelatin benchmark capsules begin to show considerable stress damage after only one pin roll. On the other hand, the gelatin capsules of the present invention remain visually intact and keep their original shape.

Mechanical force represented by 5 pin rolls result in most gelatin benchmark capsules being broken. Under the same conditions, a good number of the gelatin capsules of the present invention are broken, although almost half are still intact.

All gelatin benchmark capsules are broken after the application of a force represented by 10 pin rolls. A similar result is observed with the gelatin capsules of the present invention.

Shear obtained by sliding the top slide over the bottom results in complete breakage of both benchmark and invention capsule samples.

The test demonstrates that the gelatin capsules of the present invention are more resistant to mechanical stress and have a better breakability profile than the benchmark capsules.

## Claims

1. A microcapsule composition comprising a plurality of microcapsules, each microcapsule comprising a core material encapsulated in a shell, wherein the shell comprises a complex coacervate formed from at least two oppositely charged colloids, one of which is a protein, and wherein the protein is cross linked to a hardening agent to form amide linkages between the protein and the hardening agent.

2. A microcapsule composition according to claim 1wherein the hardening agents contains more than one anhydride group, which is reactive with amino groups on the protein to form amide cross-linkages.

3. A microcapsule composition according to claim 1 or claim 2 wherein the shell of a microcapsule comprises structural units, formed by the reaction of the protein and the hardening agent, of the following general formula 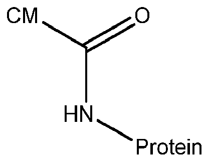 wherein
CM represents the residue of the common central moiety after the reaction of its anhydride functionality with amino functionality on a protein; and -NH-Protein is the residue of an animal or vegetable protein.

4. A microcapsule composition according to claim 3 wherein the hardening agent is a polymeric anhydride.

5. A microcapsule composition according to claim 4 wherein the polymeric anhydride exhibits an average molecular weight between 10000 and 1000000g/mol, more preferably between 10000 and 500000 g/mol and even more preferably between 10000 and 300000 g/mol.

6. A microcapsule composition according to claims 3 to 5 wherein the microcapsule shell comprises divalent structural units, formed by the reaction of the protein and the polyanhydride hardening agent, having the following general formula 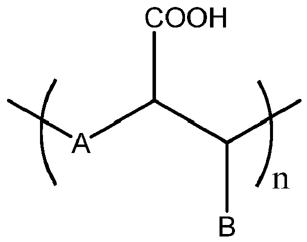 wherein A is a divalent moiety comprising 1 to 20 carbon atoms, which may be aliphatic, cycloaliphatic or aromatic; B is an acid or an amide group linked to the protein residue, more particularly an animal or vegetable protein, for example gelatine, more particularly fish gelatine; and n is an integer greater than 1.

7. A microcapsule composition according to claim 3 to 6 wherein the microcapsule shell comprises divalent structural units, formed by the reaction of the protein and the polyanhydride hardening agent, having the following general formula 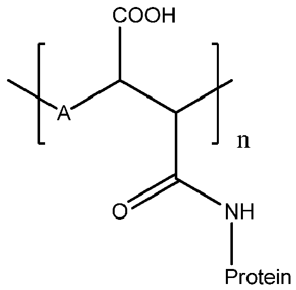 wherein A is a divalent moiety comprising 1 to 20 carbon atoms, which may be aliphatic, cycloaliphatic, or aromatic; more particularly the divalent moiety A is a group -(CRR')ₘ-, wherein R and R' are independently selected from hydrogen, methyl, or higher alkyl and m is 0, 1 or higher integer; n is an integer greater than 1 and -NH-Protein is a residue of an animal or vegetable protein, for example gelatine, more particularly fish gelatine.

8. A microcapsule composition according to any of the preceding claims wherein the hardening agent is selected from the group consisting of 3,3',4,4'-benzophenonetetracarboxylic dianhydride; 1,2,4,5-benzenetetracarboxylic dianhydride; 1,4,5,8-naphthalenetetracarboxylic dianhydride; and 3,4,9,10-perylenetetracarboxylic dianhydride; bicyclo[2,2,2]octo-7-ene-2,3,5,6-tetracarboxylic dianhydride; ethylenediaminetetraacetic dianhydride; diethylenetriaminepentaacetic dianhydride; diethylenetriaminepentaacetic dianhydride; ethylenediaminetetraacetic dianhydride; bicyclo[2.2.2]oct-7-ene-2.3.5.6-tetracarboxylic dianhydride; cyclobutane-1,2,3,4-tetracarboxylic dianhydride; 3,3',4,4' biphenyltetracarboxylic dianhydride; and perylene-3,4,9,10-tetacarboxylic dianhydride; poly(ethylene-maleic anhydride); poly(maleic anhydride-1-octadecene); poly(styrene-co-maleic anhydride); poly(methyl vinyl ether-maleic anhydride); poly(ethylene-co-ethyl acrylate-co-maleic anhydride); poly(ethylene-co-vinyl acetate)-graft-maleic anhydride; polyethylene-graft-maleic anhydride; and polypropylene-graft-maleic anhydride; Poly(azelaic anhydride); poly(isobutylene-alt-maleic anhydride); poly(styrene-alt-maleic anhydride); poly(trimethylolpropane/di(propylene glycol)-alt-adipic acid/phthalic anhydride); poly[(isobutylene-alt-maleimide)-co-(isobutylene-alt-maleic anhydride)]; polystyrene-bloc-poly(ethylene-ran-butylene)-block-polystyrene-graft-maleic anhydride; and poly[(isobutylene-alt-maleic acid, ammonium salt)-co-(isobutylene-alt-maleic anhydride)]

9. A microcapsule composition according to any of the preceding claims **characterised in that** is free of formaldehyde, glyoxal or glutaraldehyde.

10. A process of forming a microcapsule composition comprising the steps of
I) forming a dispersion of tiny droplets of core material in a coacervate of oppositely charge colloids, one of which colloids is a protein, and forming a coating of coacervate around the droplets;
II) gelling the coacervate coating by reducing the temperature below the gel temperature of the coacervate to formed gelled microcapsules; and
III) hardening the gelled microcapsules by the addition of a hardening agent that reacts with the protein to form amide cross-linkages.

11. A process according to claim 10 comprising the steps of
i) creation of an oil-in-water emulsion comprising a dispersion of tiny droplets of core material in an aqueous mixture of at least two oppositely charged colloids, one of which is a protein;
ii) applying a phase-inducing agent to the emulsion to cause the colloids to coacervate and condense around the droplets to form a liquid coacervate coating around said droplets;
iii) gelling the coacervate coating by reducing the temperature below the gel temperature of the coacervate to formed gelled microcapsules; and
iv) hardening the gelled microcapsules by the addition of a hardening agent that reacts with the protein to form amide cross-linkages.

12. A process according to claim 10 or claim 11 wherein the process is monitored by pH changes, and the reaction is terminated when the pH of the aqueous phase becomes constant over time.
